# EUROPEAN PATENT APPLICATION

(11) **EP 3 524 284 A1**
(43) Date of publication of application: **14.08.2019**
(21) Application number: 18156152.3
(22) Date of filing: 09.02.2018
(51) Int. Cl.: A61M 1/10, A61F 2/24, A61M 1/12, H01L 41/00

(54) **IMPLANTABLE DEVICE AND CONTROL METHOD**

(71) Applicant: Koninklijke Philips N.V., 5656 AE Eindhoven (NL)
(72) Inventor: HENDRIKS, Cornelis Petrus, 5656 AE Eindhoven (NL); JOHNSON, Mark Thomas, 5656 AE Eindhoven (NL); KOLEN, Alexander Franciscus, 5656 AE Eindhoven (NL); VAN LIESHOUT, Marjolein Irene, 5656 AE Eindhoven (NL); HILGERS, Achim, 5656 AE Eindhoven (NL); VAN DER HORST, Arjen, 5656 AE Eindhoven (NL)
(74) Representative: de Haan, Poul Erik

(57) **Abstract**

An implantable device (12) comprises an EAP actuator and a sensing means. The sensing means is configured to monitor a force external to the device acting in a direction either with or counter to a direction of actuation of the actuator, and a controller is adapted to control the actuator to actuate at a moment when force counter to the direction of actuation is sensed to be lowest within a given monitoring window or force with the direction of actuation is sensed to be at its highest within a given time window. In this way actuation is effected at a moment of least resistance force, reducing the power needed for deployment of the actuator, and permitting actuation to occur even in conditions experiencing large variable forces.

## Description

### FIELD OF THE INVENTION

This invention relates to an implantable device, in particular an implantable device comprising an electroactive polymer actuator.

### BACKGROUND OF THE INVENTION

There is an unmet clinical need for accurate, unobtrusive and long term monitoring of patients with chronic diseases such as heart failure, peripheral artery disease or hypertension. The purpose of monitoring is to provide reassurance or early warnings, or to reduce or to control medicine use. Wearable or skin insertable devices do not serve this need since they do not have direct access to the cardiovascular system. To meet this clinical need, smart implantable devices are needed. In general, "smart" may refer to the integration of sensors and actuators. These may include for instance blood pressure sensors (Cardiomems), restenosis sensors (Instent), or actuators for controlled drug delivery such as a micro-peristaltic pump (MPS microsystems).

There is also a need for smart implantable devices capable of interacting within internal bodily elements, for instance to manipulate them for a clinical purpose, or to perform a sensing function associated with the element by acting against the element.

Responsive materials, in particular electroactive polymers (EAP) enable soft, silent and low power sensors and actuators in a small form factor. Because of these benefits, EAPs are envisioned to work as artificial muscles in the human body. Some examples of potential in-body applications with EAPs are: provision of a heart patch offering controlled drug delivery; heart-assist devices (e.g. for assisting in contracting an atrium or ventricle); artificial sphincters and peristaltic conduits, e.g. urinary or oesophageal; rehabilitation of facial movement in patients with paralysis, e.g. eyelid blinking.

In many cases the actuators of implantable devices may need to operate under conditions where varying and high forces are present. Examples are operation in the heart, in moving or pulsating arteries, against respiratory motion, or in sphincter muscles. In these cases, the strong forces can overwhelm the actuator forces, rendering the devices ineffectual or at least unreliable.

There is a need therefore for an improved means of controlling implantable devices which enables them to operate effectively and reliably in conditions where varying and high forces are present.

### SUMMARY OF THE INVENTION

The invention is defined by the claims.

To solve the above stated problem, it has been considered by the inventors to synchronize actuation with muscle movements or blood pressure cycles so that actuators are not working against strong counter forces.

One approach considered by the inventors was to synchronize EAPs using human electrophysiological signals, e.g. ECG. A disadvantage of this however is there is a delay between the electrical activity of the heart and the mechanical muscle activity. Algorithms are therefore needed to account for this delay and to synchronize the motion. This makes devices more complex, and also less versatile, since they only work under the particular conditions and for the particular applications for which the algorithms have been programmed.

An improved solution is therefore required.

According to an aspect of the invention, there is provided an implantable device comprising:
a support structure;
an actuator comprising an electroactive polymer material, the actuator being mounted to the support structure and wherein the actuator has a direction of actuation;
a sensing means adapted to sense an external force being exerted in a direction opposing said direction of actuation or in said direction of actuation;
a controller for controlling actuation of the actuator and receiving signals from the sensing means, the controller adapted to:
   interpret signals from the sensing means to monitor said force over time; and
   drive the actuator to actuate at a moment in time when force opposing the direction of actuation is sensed to be at its lowest within a given time window or force in the direction of actuation is sensed to be at its highest within a given time window.

The implantable device of the invention actively senses environmental forces and times actuation so as to coincide with a moment of relative low or minimal resistance force and/or relative high contributory force. In this way, the actuator is not required to work against strong counter forces, and/or takes advantage of forces which are working with the actuator directionality.

This provides a simple solution, allowing accurate synchronization with body motions, but without requiring the use of slow interpretive algorithms. This is because the approach of the invention is based on directly sensing environmental forces, and timing actuation accordingly.

In examples, the implantable device may be for exerting a force against an internal bodily element. The actuator may facilitate this. In other examples, the implantable device may be for performing a sensing function. The actuator may be for deployment or control of the sensor or for implementation of the sensing. In other examples, the device may be for providing a prosthetic valve or other element. The actuator may be for adjusting a size or fit of the element.

The sensing means is adapted to sense an external force being exerted in a direction toward the actuator, and in particular in a direction opposing a direction of actuation or in the direction of actuation. This may in examples be a force being directly applied to the actuator (or a part of it), or may be a force being exerted by an element separated from the actuator, but which the actuator is configured in use to apply force against. In the latter, case, it is desirable to sense the force being exerted by said element before deploying the actuator, to ensure that deployment is timed to coincide with a moment of low force exerted in a direction toward the (at that time non-deployed) actuator.

Additionally or alternatively, sensing the force being exerted may comprise sensing a force being applied to at least a region of the actuator itself.

The actuator has a direction of actuation, and wherein said external force is a force being exerted in a direction opposing said actuation direction (i.e. counter to said actuation direction) or in a direction in (i.e. co-directional with, or directionally correspondent with) the direction of actuation. In this way, the sensed force relates directly to a resistance force or a contributory force which the actuator will experience upon actuation.

By way of example, and as will be explained below, the device may comprise an adaptive diameter ring for extending around a blood vessel for circumferentially compressing the vessel on actuation. In this case, the sensor may be adapted to sense a force being exerted in a direction opposing the direction of actuation by the vessel, for instance in a radially outward direction onto the ring. In other examples, the device may comprise an artificial valve for positioning inside a blood vessel and being adapted to reduce in diameter upon actuation, for assisting in fitting of the vessel. Here, the sensing means may be adapted to sense a force exerted by the blood vessel in a direction which is in the direction of actuation, e.g. radially inwardly onto the outside of the ring.

The external force may in some examples be periodic, and wherein the time window is a single cycle period of the periodic force. This provides a natural and convenient time scale over which to assess force strength, to find a moment of minimal or maximal force.

The actuator comprises an electroactive polymer (EAP).

An electroactive polymer actuator may for instance comprise a material body comprising electroactive polymer (EAP) material, the EAP material being deformable in response to electrical stimulation.

By way of example, the actuator may comprise an ionic polymer membrane sensor-actuator. These are low voltage devices suitable for in-body operation.

Electroactive polymer material actuators have the advantage of mechanically simple construction and functionality. This contrasts for instance with mechatronic or other electromechanical actuators or sensors. EAPs also allow small form factor, ideal for deployment in or around small bodily structures, such as blood vessels or heart chambers, where avoiding occlusion for instance is important. They also have long lifetime, limiting the need for future invasive procedures to replace the device.

The actuator may in some examples be a sensor-actuator, the sensor-actuator providing the sensing means. This may be implemented by applying a high frequency AC (sensing) signal superposed atop a lower frequency or DC driving signal to the electroactive polymer (EAP) of the actuator This method of driving permits simultaneous sensing and actuation using the EAP actuator. This is described in detail in WO 2017/036695.

Additionally or alternatively, the sensing means may comprise a sensor element, mounted to the support structure. In this case, a separate element for performing sensing is provided. This may be a force gauge or pressure sensor for instance.

In any example, the sensing means may sense a parameter indicative of force, or may sense or measure force directly.

In examples, the implantable device may be for exerting a force against an internal bodily element. The actuator may facilitate this.

The internal bodily element may for instance be an organ or vessel or other solid structure within the body. Alternatively, the internal bodily element may be blood within a blood vessel, for instance wherein the device is for manipulating a blood flow through the vessel.

The device may be for exerting a force to manipulate the internal bodily element, for instance to adjust a dimension of the element, or to control or shape or adjust a fluid flow through a conduit or chamber. Alternatively, exerting a force may for instance be for deploying the actuator against the element for performing a sensing function, for instance to deploy the actuator into a blood flow path to sense blood flow or blood pressure or another parameter.

The sensing means may be adapted in use to sense an external force being exerted in a direction toward the actuator by said internal body element. This ensures that the sensed force pertains to a resistance or contributory force being exerted by the bodily element upon the actuator.

In some examples, the actuator may be arranged to adjust a dimension of said internal bodily element (by means of the force exerted upon it). For instance, this may comprise adjusting an internal dimension e.g. an internal diameter or volume, of a blood vessel, or an internal volume of a heart chamber for instance (e.g. for assisting in pumping blood from the chamber).

In some examples, the actuator may be for positioning within a bodily chamber or conduit, and is arranged in use to permit manipulation of a fluid flow through said chamber or vessel. For instance, the actuator (and typically also the device) may be for positioning within a blood vessel, and arranged in use to permit manipulation of a blood flow through said vessel. In this case, the internal bodily element is the fluid (e.g. blood) within said chamber or conduit (or vessel).

In examples, at least a part of the actuator may be adapted in use to rest against said internal bodily element (e.g. against which a force is to be applied), and wherein the sensing means is adapted in use to sense a force exerted by the bodily element on the actuator. This provides a convenient arrangement for monitoring force exerted since the element is arranged in contact with the device and hence the sensing means. This arrangement may be suitable in the case for instance of a device for adjusting the dimension of the element.

In more particular examples, at least a part of the actuator may be adapted in use to rest against the internal bodily element when in a non-deployed position, and wherein the sensing means is adapted in use to sense a force exerted by the bodily element on the actuator when in said non-deployed position. This provides a convenient arrangement for monitoring force in advance of deployment.

In accordance with one set of examples, the device comprises an adaptive diameter ring for adjusting an internal dimension of an internal bodily element (or structure), the actuator being arranged such that actuation of the actuator changes a diameter of the ring for effecting said adjustment.

In particular examples, the adaptive diameter ring may comprise an annular arrangement of actuators which at least partially define the ring, the actuators being adapted to deform in a radial direction upon actuation to thereby adjust the diameter of the ring, and optionally wherein said external force is a force exerted toward the actuators in a radial direction. The force may be a force exerted toward the actuators in an opposing radial direction (to the direction of deformation of the actuators).

In certain examples, the ring may be for positioning around the outside of the internal bodily element of structure. In this case, the sensed external force may be an external force in a radial outward direction.

In certain other examples, the ring may be for positioning within the interior of a bodily element, e.g. in the interior of an annulus of the heart, as part of a prosthetic heart valve. In this case, the sensed external force may be an external force in a radial inward direction.

In accordance with one set of examples, the adaptive diameter ring may be for extending around a blood vessel, for adjusting an internal dimension of the blood vessel. The internal dimension may be an internal diameter for instance, or a circumference, or a cross-sectional area, or a volume.

In this set of examples, the sensing means may be for sensing a force exerted in a direction radially outward of the vessel, by blood within the vessel or by a wall of the vessel.

In accordance with a further set of examples, the ring may be for positioning around a chamber of a heart for adjusting in use an internal dimension of said chamber. The internal dimension may be an internal volume for instance, or a diameter.

In this set of examples, the sensing means may be adapted to sense a force exerted in a direction outward of said chamber, for instance by a wall of the chamber (or a muscle within the wall for instance).

In accordance with one or more examples, the device may comprise a prosthetic valve for a blood vessel or for the heart, the adjustable diameter ring forming at least part of an outer radial wall of said valve.

In accordance with any example of the invention, the actuator may be a bi-stable actuator. Bi-stable means that the actuator is drivable between at least two stable actuation positions through application of a drive signal, the actuator being adapted to remain in each of said stable positions upon removal of the drive signal.

Examples in accordance with a further aspect of the invention provide a method of controlling an implantable device, the implantable device comprising
a support structure,
an actuator comprising an electroactive polymer material, the actuator being mounted to the support structure and wherein the actuator has a direction of actuation;
a sensing means adapted to sense an external force being exerted in a direction opposing said direction of actuation or in said direction of actuation;
a controller for controlling actuation of the actuator and receiving signals from the sensing means, the controller adapted to:
the method comprising:
   interpreting signals from the sensing means to monitor said force over time; and
   driving the actuator to actuate at a moment in time when force opposing the direction of actuation is sensed to be at its lowest within a given time window or force in the direction of actuation is sensed to be at its highest within a given time window.

### BRIEF DESCRIPTION OF THE DRAWINGS

Examples of the invention will now be described in detail with reference to the accompanying drawings, in which:
Fig. 1 shows an example implantable device according to an embodiment;
Fig. 2 shows a further example implantable device according to an embodiment, comprising an adaptive diameter ring;
Fig. 3 shows a graph illustrating the timing of a step-wise adjustment of the diameter of an implantable device relative to the external force being applied to the device;
Fig. 4 shows a further example implantable device according to an embodiment, comprising an adaptive diameter ring;
Fig. 5 shows a further example implantable device according to an embodiment, comprising an adaptive diameter ring;
Figs. 6 and 7 illustrate an example adaptive diameter ring for implementation in embodiments of the invention;
Fig. 8 shows a further example implantable device according to an embodiment, comprising a heart-assist device;
Fig. 9 illustrates timing of activation of the device of Fig. 8 relative to external force exerted on the device; and
Fig. 10 shows a further example implantable device in accordance with an embodiment, comprising a pre-tensioned ring.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an implantable device comprising an EAP actuator and a sensing means. The sensing means is configured to monitor a force external to the device acting in a direction either with or counter to a direction of actuation of the actuator, and a controller is adapted to control the actuator to actuate at a moment when force counter to the direction of actuation is sensed to be lowest within a given monitoring window or force with the direction of actuation is sensed to be at its highest within a given time window. In this way actuation is effected at a moment of least resistance force, reducing the power needed for deployment of the actuator, and permitting actuation to occur even in conditions experiencing large variable forces.

As discussed above, the invention is aimed at solving the problem of reliably operating actuators of implantable devices in conditions where strong and variable forces are present.

To illustrate the problem more clearly, some example applications for implantable devices in accordance with the invention will now be discussed, whereby the particular environmental forces which may be experienced are described.

A first example involves placement of collapsible prosthetic heart valves. In catheter-based heart valve replacement procedures it is required to deliver, position, fit, anchor and seal the heart valve accurately in the annulus of the aorta or ventricle. Improper fitting may lead to complications such as migration, leakage or scar formation due to excessive radial forces on the tissue.

It can be difficult to determine in advance the precise required size of the heart valve. This is because of unknown calcification levels of the old tissue, which determine the mechanical deformability of the annulus, as well as person to person variations of the annulus size. Provision of a prosthetic heart valve having an adaptive outer diameter would make the placement easier and also enable a good long term performance (e.g. seal).

A means of implementing this is to incorporate an actuator in the ring of the heart valve which is operated in synchrony the beating heart muscles and with other high forces. In particular, the maximum forces exerted on the outer diameter of a prosthetic mitral valve can be as high as 6-8 Newtons during mid-systolic points, and the corresponding variation in outer diameter of the valve may be up to 40 micrometers (for nominal diameter of 29 mm).

In general, the forces generated in the myocardium are highly variable during the cardiac cycle. For example, the differences between systolic and diastolic forces are a factor 6 to 7. An adaptive diameter heart valve which is configured to actuate with, rather than against such high forces would clearly improve reliability of performance and potentially reduce the maximum actuation power required for the device.

A second example involves repair of mitral valve insufficiency. A known problem is improper closure of the Mitral or Tricuspid valve due to an enlarged annulus. A known surgical solution is to tighten the annulus with a fixed length wire. A self-adapting ring which changes its diameter (per heart cycle) would be a better solution. However, again, actuation is rendered greatly more efficient if size adjustment is performed at moments of low blood flow or pressure, so that the force required to adjust the valve size is reduced.

A third example relates to cuffs for placement in or around arteries or veins to restrict, control or support (e.g. enhance) blood flow. A number of clinical problems exist in which blood flow manipulation with a vascular cuff could solve or relieve the problem.

By way of example, in the case of left ventricle heart failure, excess blood may often be pumped into the lungs by the right ventricle, due to which fluid builds in the lungs. This problem may be relieved by clamping the vena cave and in this way restricting the blood flow into the right ventricle (to restore the heart balance).

By way of a further example, after the treatment of peripheral artery disease with a stent, vascular steal can occur. Vascular steal is a negative effect upon cardiovascular circulation which can occur following a local treatment applied to one part of the cardiovascular system. In particular, opening a diseased artery with a stent locally increases the blood flow, but as a consequence blood flow in other arteries may decrease. It can be very difficult to predict.

This problem may be relieved by providing a stent whose size is adjustable after placement (should this become necessary) in order to control the blood flow. Here, due to the highly variable outer pressure exerted on the stent by blood flow, it would be beneficial to time size adjustments to coincide with moments of low blood pressure, i.e. low force in a radial direction.

In a further example, ischemia in the lower legs or feet, due to poor circulation in arteries or capillaries, can lead to a diabetic foot or chronic limb ischemia (CLI). One of the potential causes is insufficient blood pressure. The blood pressure in the arteries of the lower leg may be reinforced by supporting the blood flow, e.g. with a vascular cuff based peristaltic pump. In this case, in order to assist blood flow, it is important that contractions of the pump are synchronized with blood flow rhythm. In particular, the pump should contract (actuate) as the pressure reaches its lowest point in the blood vessel (to assist when blood is draining).

Faulty valves and/or dilated leg veins can create pooling and extravasation of blood in the leg, leading to swelling and/or thrombosis. The problem may be relieved by, again, supporting the blood flow, for example with a vascular cuff based peristaltic pump.

The present invention proposes to mitigate the effects of strong environmental forces by synchronizing actuation according to said forces. The invention in particular proposes to do this by timing actuation to coincide with a moment of lowest force.

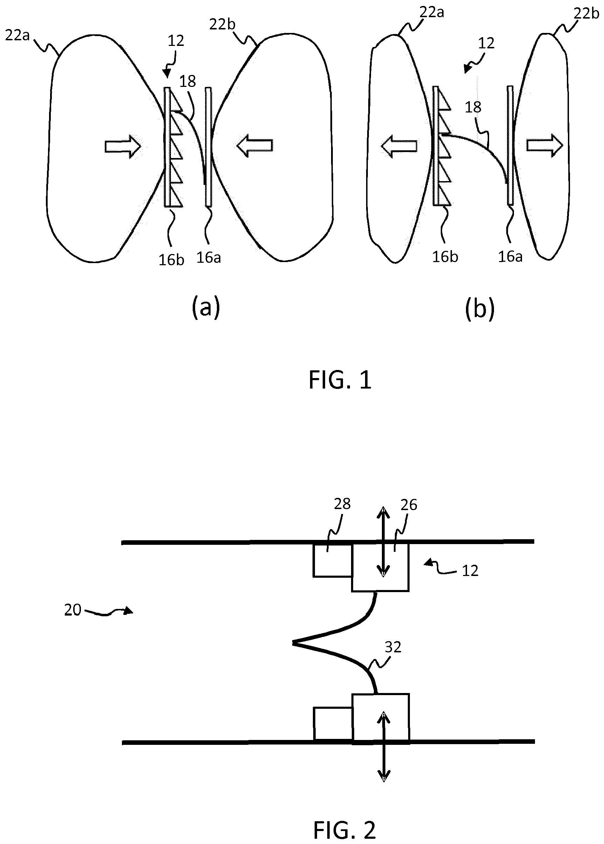

The basic concept of the invention is illustrated schematically in Fig. 1. An implantable device 12 comprises a support structure 16a, 16b, and an actuator having an actuation element 18 comprising an electroactive polymer material. The actuator element in this example is mounted to a fixation element 16a of the support structure, and is arranged to extend outward toward a retaining element 16b comprising a series of notches adapted to engage with the end of the actuator element to retain it in a fixed position.

The implantable device is shown implanted in a body, positioned between internal bodily elements in the form of a pair of muscles 22a, 22b which exhibit co-operative flexing action. The EAP actuator 18 is a sensor-actuator, adapted to provide simultaneous force sensing and actuation (see below for greater detail). Fig. 1(a) shows the arrangement at a moment when the muscles are contracted. Fig. 1(b) shows the arrangement when the muscles are relaxed.

The sensor-actuator is adapted to sense a force exerted by the muscles on the sensor-actuator. The arrangement is such that the force exerted is in a direction against the direction of actuation of the actuator element 18.

A controller (not shown) is adapted to interpret sensing signals received from the sensor-actuator and monitor the force exerted by the muscles 22a, 22b upon the actuator element 18 over time. When adjustment of the actuator position is required, the controller is adapted to monitor the force, and actuate the actuator to move to the new position at a moment in time when the force is sensed to be at its lowest within a given time window. For instance, the muscles may be respiratory muscles or heart muscles, such that the muscles exhibit periodic flexing behavior. The force in this case exerted upon the actuator sensor 18 is a periodic force. The controller may be adapted to actuate the actuator at a moment of lowest sensed force within a given cycle period of the periodic force.

Fig. 1(b) shows actuation of the actuator 18 at such a moment of lowest force. This occurs at a time when the muscles 22a, 22b are relaxed, thereby exerting least force in the direction of the actuator. The actuator is actuated by the controller, causing it to deform in such a manner as to shift to a lower of the series of notches of the retaining element 16b. The actuator is hence controlled to actuate at a moment of least force resistance from the muscles.

The new actuation position moves the actuator to more extended position. Since the actuator is locked in position by the retaining element 16b, the implantable device thereby fixes a minimum spacing between the muscles during subsequent flexing. The implantable device is thus arranged in use to exert a force upon the muscles during use, against the natural flexing action of the muscles, thereby maintaining the spacing between them. This may be useful in practical applications for instance to maintain a minimum flow path for a bodily fluid in cases for example where the muscles are functioning incorrectly and causing partial occlusion of the passage between them.

The device (in accordance with any embodiment) may be powered through either a wired or wireless power supply which may be comprised as part of the device or may be separate to it. Examples will be described in greater detail below.

The actuator 18 comprises an EAP actuator. In accordance with any embodiment of the present invention, EAP actuators can be provided in different configurations, for different actuation behavior.

In a simplest configuration, the actuator may comprise an electroactive polymer layer sandwiched between electrodes disposed on opposite sides of the electroactive polymer layer. A voltage is applied across the EAP layer by the electrodes to cause the EAP layer to expand in all directions, in-plane with the layer.

In various examples of the present invention (including the example of Fig. 1), it is required that deformation of the actuator occur in only a single direction, or mainly in a single direction. In this case, the structure described above is supported on a carrier layer. When a voltage is applied across the EAP layer using the electrodes, the whole layer structure is caused to curve (as illustrated in Fig. 1(b)) or to bow. Bowing behavior can be achieved by clamping the two ends of the layer structure. When the layer is deformed, the restriction of its ends forces the deformation in an out-of-plane direction, in a bowing action.

The nature of this movement for example arises from the interaction between the active layer which expands when actuated, and the passive carrier layer. To obtain asymmetric curving around an axis, molecular orientation (film stretching) may for example be applied, forcing the movement in one direction.

The expansion in one direction may result from the asymmetry in the EAP polymer, or it may result from asymmetry in the properties of the carrier layer, or a combination of both.

An electroactive polymer structure as described above may be used both for actuation and for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured.

Simultaneous sensing and actuation (in accordance with any embodiment of the invention) can be achieved by measuring the impedance of the outer electrodes separately to the actuation voltage. The impedance provides an indication of force applied to the actuator. Alternatively, it may be achieved through applying a driving scheme in which a high frequency, relative low amplitude, AC signal is applied superposed with an underlying higher voltage actuation drive signal. The drive signal may be a DC signal or relative low frequency AC signal. This driving scheme for achieving simultaneous sensing and actuation is described in detail in WO 2017/036695.

In accordance with any embodiment of the invention, the EAP actuator may be a bi-stable or multi-stable EAP actuator. By this is meant that the actuator is drivable between two or more stable actuation positions through application of a drive signal, whereby the actuator is adapted to remain in each of the stable positions upon removal of the drive signal. This means that subsequent contraction of the muscles 22a, 22b will not be able to deform the actuator away from each stable actuation position, once set. Use of bi-stable EAP actuators is described in WO 2016/193412, and the teachings therein may be applied to implement bi-stable actuation in any embodiment of the present invention.

Although a sensor-actuator is used in the example of Fig. 1, in other examples, a separate sensor element may instead be used to sense a force exerted in a direction toward the actuator 18. The sensor element may by way of example comprise a pressure-sensitive film applied to a surface of the EAP actuator. The sensor element may be adapted to sense force directly, or to sense a parameter indicative of force, e.g. pressure, or even a voltage signal.

Fig. 1 shows a simple first example implantable device in accordance with the invention, for purposes of illustrating the concept of the invention. Features and properties described in relation to this simple example are applicable widely across all particular embodiments of the invention.

As can be recognized from the previous discussion, the concept of the invention can be implemented in wide variety of different particular applications. To illustrate the invention, a number of example embodiments of the inventive concept will now be described with reference to the drawings. Each embodiment is to be understood as exemplary only; the underlying inventive concept is applicable across a broad range of different particular implementations.

In accordance with a first set of example embodiments, the implantable device may comprise an adaptive diameter ring for sealing an annulus or adjusting the diameter of a bodily tube or conduit. In particular, the implantable device may be for optimizing the outer diameter of a prosthetic heart valve (for ensuring optimal issue contact pressure for optimal sealing), for remedying a dilated annulus in the heart, or for adapting the inner diameter of a vascular cuff in order to adapt the blood flow.

Fig. 2 schematically depicts an example implantable device 12 comprising a prosthetic heart valve and being adapted for optimizing a diameter of the valve. The implantable device comprises an adaptive diameter ring 26 comprising an arrangement of one or more actuators for adjusting the diameter of the ring. Curling longitudinally outward and radially inward from a circumferential periphery of the ring is a pair of prosthetic valve leaflets 32, which meet in a sealing fashion at a radially central point, longitudinally displaced from the ring. The leaflets mutually seal against one another, to thereby seal the valve.

The implantable device 12 further comprises a controller 28, which also comprises a power supply for the device.

The device is implanted in an artery 20 of the heart. The actuator(s) comprised by the adaptive diameter ring are controllable by the controller 28 to actuate in order thereby to adjust a diameter of the ring. The actuator(s) may be arranged such that actuation increases a diameter of the ring, or may be arranged such that actuation decreases a diameter of the ring. There may be provided two sets of actuators being configured with different actuation directionality, such that actuation of one set induces diametric increase of the ring 26 and actuation of the alternate set induces diametric reduction of the ring.

The actuators of the adaptive diameter ring 26 in this example are sensor-actuators. However, alternatively, a separate sensor element may be provided (not shown), for instance mounted to the adaptive diameter ring for making contact with the artery 20 wall.

Where the actuators are configured for reducing a diameter of the ring, the sensor-actuators are adapted to sense a force exerted by the artery wall in a direction in the direction of actuation (i.e. radially inwardly). Where the actuators are configured for increasing a diameter of the ring, the sensor-actuators are adapted to sense a force exerted by the artery wall in a direction opposing the direction of actuation (i.e. radially outwardly). The sensor-actuators are preferably configured to sense forces in both directions, such as to facilitate actuation either radially inwardly or outwardly.

In use, after implantation, it may be beneficial to adjust a diameter of the ring 26 to better secure and seal the artificial valve within the artery 20. This may be performed by actuating the actuators of the ring 26 to slightly expand the diameter of the ring, to ensure the ring is pressed firmly against the wall of the artery 20, or to slightly reduce the diameter to ensure the ring is not over-stretching the artery wall.

Optimizing the sealing can be performed based on time-average radial force exerted on the ring by the artery 20 wall or for instance maximal force exerted by the wall in a given cycle. Optimal sealing may have a known (average or maximal) radial inward force associated with it (i.e. when sealing is optimal, the pressing force between the ring and the artery wall is known to be at a particular level). The ring diameter may simply be adjusted until this known optimal radial force is achieved.

The adjustment may be step-wise adjustment. This may involve following an adjustment control loop, wherein sensing signals are monitored to detect an average radial force upon the ring. If the sensed average force differs from the known optimal force for optimal sealing by a certain threshold amount, a step-wise change in the ring diameter is performed by appropriately actuating the actuator(s) of the ring. The average radial force the ring is then re-sensed, to determine if deviation from the optimal force is still present. If so, another step-wise diameter adjustment is performed. The process is repeated until the optimal radial force is reached.

Due to the blood pumping through the artery 20, pressure within the artery varies periodically with the pulsing of the heart. This changes the force being exerted by the artery wall upon the ring 24 over the cycle. It may be preferable to actuate the ring at a moment when the wall is exerting least radial force in a direction opposite to a direction of intended actuation, so that adjustment of the ring is acting against the smallest resistance force. Where it is intended to expand the ring, this corresponds with a moment of highest blood pressure in the artery, since at this point the pressure of the blood assists in pushing out the artery wall, relieving radial inward force being exerted on the ring. If it is intended to constrict the ring, this corresponds with a moment of lowest blood pressure, since at this point, the natural inward radial resistance force of the wall assists in pushing the ring to a smaller diameter.

To this end, the controller 28 is adapted to interpret sensing signals from the sensor-actuators of the ring 26 and to monitor a force being exerted upon the sensor-actuator over time by the artery 20 wall. When adjustment of the diameter is desired, the controller 28 is adapted to identify a moment of lowest force within a periodic cycle of the exhibited force, and to control the actuators to actuate at this moment.

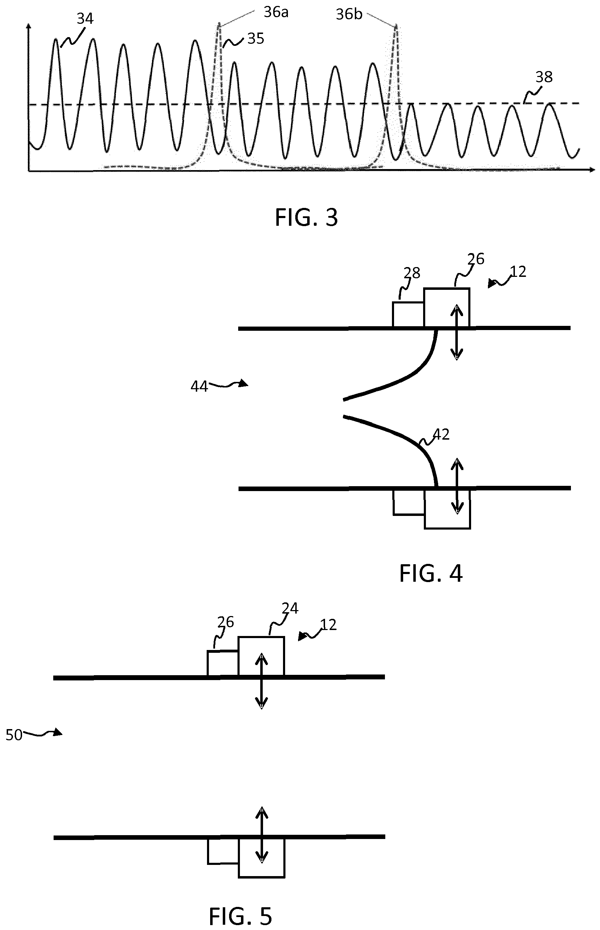

The operation is illustrated by the graph of Fig. 3 which shows radial force (y-axis) sensed by the sensor-actuator(s) of the adaptive diameter ring 26 as a function of time (x-axis). Line 34 shows sensed force over time. Line 35 shows force exerted by the actuator(s) of the ring for adjusting the diameter. Line 38 illustrates the desired maximal radial force level for the ring in order to achieve optimal sealing.

It can be seen from the graph that the radial force oscillates in a periodic fashion. This is due to the varying pressure in the artery 20 caused by the beating of the heart. The maximal force is initially too high. The controller therefore effects a first stepwise adjustment in the ring diameter. This is effected by actuating the actuator(s) to change (in this case reduce) the diameter. The first actuation event, for the first step-wise diameter change is shown by peak 36a. The controller times the actuation to coincide with a moment of maximal (inward) radial force over the given cycle. A moment of maximal force is chosen because the radial inward force in this case is in a direction with the direction of actuation of the actuators (i.e. radially inward).

The actuation and resulting diameter change reduces the average (and maximal) force being exerted upon the ring by the artery wall. However, the force is still higher than the optimal force 38. A second step-wise adjustment in the diameter is therefore performed by actuating the actuator(s) a second time (shown by actuation event 36b).

This step-wise adjustment reduces the maximal force to a level below the desired maximal force 38, and hence completes the optimal fit adjustment.

The implantable device may be adapted for adjustment only once, after initial implantation, to optimize fit and sealing within the artery. The power source comprised by the controller 28 may for instance be a battery power source having only enough charge to power the device for a short period after implantation.

Fig. 4 shows a second example implantable device 12 comprising an adaptive diameter ring 26. The device in this example is configured for remedying a dilated annulus in the heart, in particular for remedying mitral valve insufficiency. As illustrated in Fig. 4, mitral valve insufficiency is a fault whereby leaflets 42 of the mitral valve fail to fully seal again one another, leading to leakage. This is typically caused by dilation of the ventricle 44, which pulls the mitral valve leaflets apart.

To remedy the dilation, an implantable device 12 in accordance with an example of the invention, comprising an adaptive diameter ring 26, may be fitted around the periphery of the dilated annulus for re-configuring a diameter of the ventricle 44 at the location of the mitral valve. The ring may be similar in construction and operation to that described above in relation to Fig. 2 and comprises a controller 28 for controlling actuation of sensor-actuators comprised within the ring 26, the actuation configured to effect adjustment of a diameter of the ring.

Once the ring is installed around the location of the annulus, its diameter may be reduced, thereby countering the dilation of the ventricle and repairing the behavior of the mitral valve.

To ensure that the sensor-actuators of the ring 26 are not working against strong forces when deforming, the controller is adapted to monitor sensing signals received from the sensor-actuators and to actuate the actuators at a moment when radial outward force exerted on the sensor-actuators by the ventricle 44 wall is lowest. Due to the pulsing of blood through the ventricle, the forces are periodic with the beating of the heart. The moment of lowest force (in a given heart cycle) will coincide with a moment of lowest blood pressure (lowest blood flow) through the annulus.

Fig. 5 shows a further example implantable device 12 according to the invention, comprising an adaptive diameter ring 24 arranged around a blood vessel 50 for adjusting a diameter of the vessel. The device in this case may have the same construction as that shown in Fig. 4 and described above. The device in this case forms a vascular cuff, and through adjustment of the diameter of the ring 24 permits adaptation of the blood through the vessel 50.

As discussed above, this may be for restricting blood flow, for instance to restrict blood flow into the right ventricle in the case of left ventricle failure. It may be for supporting blood flow. For instance (as described above) if the ring is controlled to contract in diameter cyclically in synchrony with blood flow through the vessel, this can assist in pumping blood through the vessel. The device in this case forms a peristaltic pump.

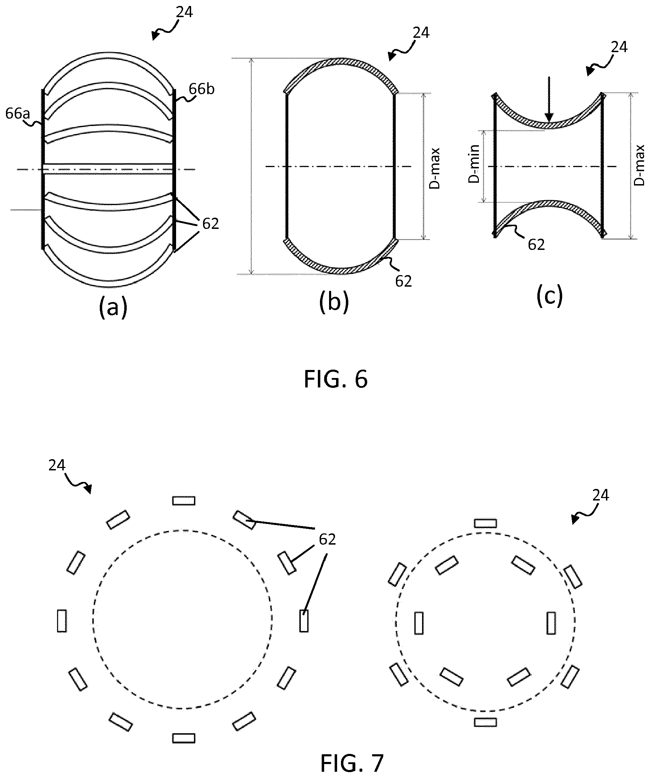

An example adaptive diameter ring 24 in accordance with the examples above is illustrated schematically in Figs. 6 and 7.

Fig. 6(a) shows a side view of the ring (facing one side of the periphery of the ring, in parallel with a radial plane of the ring). Figs. 6(b) and 6(c) show a cross-sectional view through the ring, viewed from the same side direction as Fig. 6(a). Fig. 6(b) shows the ring in a non-actuated position. Fig. 6(c) shows the ring in an actuated state. Fig. 7 shows a top-down view of the adaptive diameter ring 24.

The ring 24 comprises an annular arrangement of EAP elements (segments) 62, extending around the periphery of the ring. The EAP segments in this example are mounted to a rigid ring frame 66 which forms at least a section of a support structure of the implantable device 12. The rigid ring frame is formed of two annular portions 66a, 66b, between which the EAP segments each extend. The frame portions anchor each end of each of the EAP segments, such that upon electrical stimulation of the EAP segments, each is induced to bow radially inwardly, as illustrated in Fig. 6(c). This radial inward deflection results in exertion of a force upon any bodily structure or element situated within the annulus of the ring, which enables adaptation of the dimensions of the structure for instance (as in the examples of Figs. 4 and 5 above). Alternatively, any element coupled to the interior of the ring is displaced radially inwardly by the deflection (as in the example of Fig. 2 above).

Actuation of the ring has the effect of adjusting a diameter of the ring. The ring diameter can in this way be adjusted between a maximum diameter, D-max, to a minimum diameter, D-min.

The degree of corresponding diameter change induced in an anatomical element manipulated by the ring, such as a blood vessel or a heart ventricle, will depend upon the strength of the force applied radially inwardly by the ring when deforming, and the strength of the resistance force exerted by the bodily element against the deformation. Variation in the degree of dimensional adjustment realised in the anatomical element can be achieved by varying the amount of force applied by the ring. This can be realised in a straightforward manner by varying the number of EAP segments which are controlled to deform.

This concept is illustrated in Fig. 7. The left-hand image of Fig. 7 shows (a top-down view of) the adaptive diameter ring in a state in which all of the EAP segments are in a relaxed (non-actuated) position. The right-hand image of Fig. 7 shows the ring in a second state in which half of the EAP segments are in an actuated (radially inward) position, and half are in a relaxed (radially outward) position. The segments alternate between actuated and relaxed around the circumference of the ring. The result is that half of the maximum possible radial force applicable by the ring is applied, resulting in a diameter change of a bodily element disposed inside the ring annulus which is approximately half of the maximum diameter change which can be achieved. Activating a greater or lesser number of elements results in a correspondingly greater or lesser force applied, and consequently a greater or lesser dimensional change of the bodily element.

It will be clear to the skilled person that this principle can be extended to enable a wide variety of different levels of bodily element diameter adjustment to be effected.

The EAP segments may in accordance with advantageous examples comprise bi-stable EAP actuators. Construction and driving of bi-stable EAP actuators is described in WO 2016/193412, which teaching is applicable to embodiments of the present invention.

Each of the segments is a separate bending actuator, comprising an active EAP layer and a passive substrate layer.

The use of a segmented ring structure, rather than a single annular body of EAP has two main advantages. First, as discussed, it enables multiple stable diameter changes to be effected by varying the number of actuated segments (on-off). Secondly, it permits particularly large maximal diameter changes (since the circumference changes considerably between the D-max position and D-min position. The mutual inward bending of oppositely placed segments makes such a large maximal diameter change possible.

The invention is not limited to the particular example of Figs. 6 and 7 for the adaptive diameter ring. In further embodiments, other arrangements could be used, which might include a single annular EAP element, or for instance a circumferentially flexible ring having one or more EAP element incorporated therein and adapted to deform in-plane with the circumference of the ring, to shorten the circumference. Other arrangements might include use of swelling EAP materials such as ionic polymer gels, which if incorporated in the ring circumference would allow reduction of the ring diameter as the material swells.

In accordance with a further example embodiment, an implantable device is provided for performing a heart assistance function (a heart assist device). The heart assist device provides an artificial muscle function for the heart, wrapping around a ventricle of the heart and contracting in synchrony with natural contraction of the heart to assist in the pumping of blood.

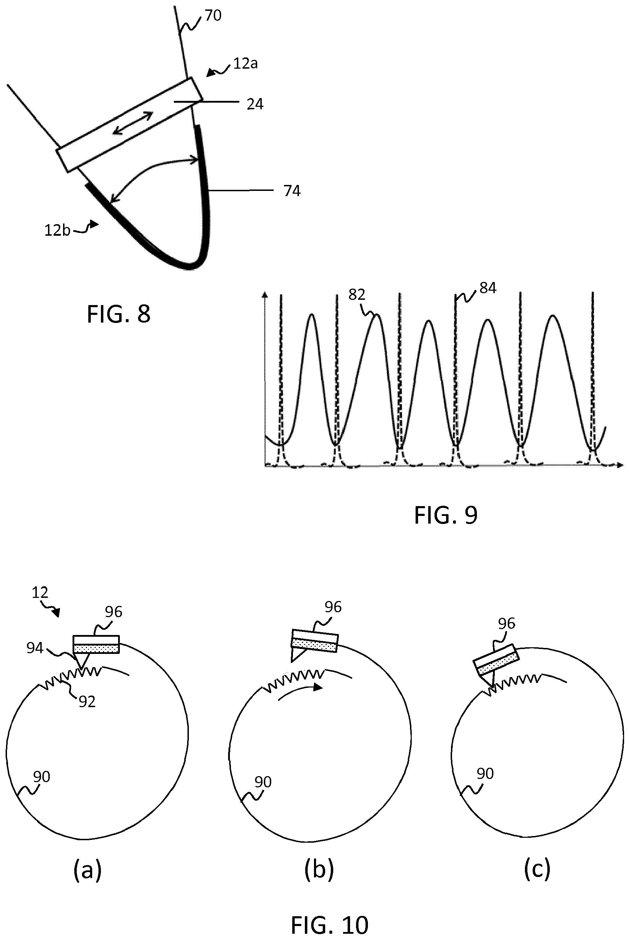

Two examples of this embodiment are illustrated schematically in Fig. 8.

The first example implantable device 12a comprises an adaptive diameter ring 24 adapted in use to extend around a ventricle 70 of the heart. The adaptive diameter ring may be provided in accordance with the example ring described above in relation to Figs. 6 and 7. The sensor-actuators (or a separate sensing element) comprised by the adaptive diameter ring 24 is (or are) adapted to monitor radial force exerted upon the ring by the ventricle. The force exerted will vary in an oscillatory manner with the beating of the heart.

A controller (not shown) is adapted to actuate the sensor-actuators to effect a contraction of the ring diameter at a moment of lowest radial force in a given cycle. This will coincide with a moment of the heart cycle at which the heart is maximally contracting (to evacuate blood from the heart). By activating at this time, the ring co-operatively assists in the heart contraction, and therefore in pumping blood from the heart. The ring effectively provides an additional 'kick' to displace the natural muscle of the heart further, in this way increasing the pumping capability of the muscle.

The second example implantable device 12b comprises a band or sleeve element 74 comprising one or more EAP actuators for providing the sleeve with an adaptive bending angle. By actuation of the EAP actuator(s), the bending angle of the band or sleeve element can be decreased, thereby exerting a squeezing or gripping force to at least a lower portion of the heart ventricle 70. As in the case of the first example device 12a, the actuation of the band or sleeve element 74 is timed by the controller to coincide with a moment of smallest outward force being exerted by the ventricle 70 upon the actuator(s). This moment coincides with maximum contraction of the heart. Hence the reduction in the bending angle of the sleeve or band, and the resulting squeezing action, co-operatively assists the natural heart muscle in pushing blood from the ventricle.

Fig. 9 illustrates the preferable control method in accordance with either of the example devices 12a, 12b of Fig. 8. The graph of Fig. 9 illustrates (line 82) the external force (y-axis) sensed by the sensing element or sensor-actuators of the ring 24 or the sleeve/band 74 as a function of time (x-axis). Line 84 illustrates the EAP actuator activation signal.

It can be seen that the external force 82 exerted by the ventricle varies cyclically as a function of time, as a result of the beating of the heart. The controller of the given device 12a, 12b is adapted to cyclically actuate the actuator(s) of the device at each point of lowest measured force in the cycle. This results in a periodic contraction behavior of the ring 24 or sleeve/band 74, thereby assisting the natural muscle of the heart.

In accordance with either device 12a, 12b, the EAP actuator(s) may by way of example comprise a dielectric elastomer or an Ionic polymer-metal composite (IPMC).

Fig. 10 illustrates a further example implantable device 12 in accordance with an embodiment of the invention. The device 12 comprises a constriction cuff for placement around a bodily lumen e.g. a blood vessel, for constriction of the lumen. The device comprises a pre-tensioned, open-ended ring 90 which is tensioned to naturally reduce in circumference in the absence of resisting force.

The ring 90 comprises a locking arrangement in the form of an actuator element 96 configured to engage with a retaining element 92 to secure the ring at a stable circumferential position. The actuator element 96 comprises an EAP actuator member coupled to a protruding locking member 94, which is directed toward the retaining element. The retaining element comprises a series of notches shaped to receive and engage the locking member to thereby lock the ring in place.

Fig. 10 shows operation of the device. Fig. 10a shows the pre-tensioned ring 90 of the device 12 in a first circumferential position, with the actuator element 96 engaged in the retaining element 92 to lock the ring in place. To reduce the diameter of the ring, a controller (not shown) is adapted to actuate the EAP actuator element. The actuator element is adapted to actuate in a radially outward direction as shown in Fig. 10b. This lifts the locking member 94 from the retaining element 92, thereby releasing the pre-tensioned ring. The ring is tensioned to naturally constrict in circumference. As a consequence, upon release of the actuator element 96, the ring constricts, reducing in diameter. Upon relaxation of the actuator, or upon driving the actuator to its previous position, the actuator is induced to reengage with the retaining element, thereby locking the ring at a new smaller circumference.

Since in this case, the constricting action of the device is provided only by the pretension stored in the material of the ring 90, the available contracting force is relatively low. It is therefore desirable to coincide constriction of the ring diameter with a moment of lowest external force acting radially outwardly on the ring (toward the actuator). To this end, the actuator element 96 may be a sensor-actuator, or there may be provided a sensing element coupled to the actuator element (e.g. a pressure sensitive film). The sensor-actuator or sensing element is adapted to sense radially outward force exerted upon the ring. This may be performed directly, or may be measured via a measurement of corresponding circumferential force exerted at the locking member 94 of the actuator element 96. The controller (not shown) is adapted to actuate the actuator element at a moment of lowest sensed force in a given time window.

Use of a pre-tensioned ring carries the advantage that the resulting device consumes only very low power, since the actuator is not required to exert force against the bodily element. However, the device has the constraint that it only permits one-way adjustment. Once constriction is effected, it cannot be reversed without invasive intervention.

The various examples have related to devices configured to manipulate internal bodily elements or to adjust placement e.g. of artificial implants. In accordance with further embodiments however, the device may be for providing sensing function, wherein the actuator is adapted for deploying a sensing element in or around a bodily element, for instance against a force exerted by a bodily element. For example, an implantable device may be provided for sensing a blood pressure or flow, comprising an actuating member adapted to actuate into a blood flow for sensing of a blood pressure or flow.

In accordance with any embodiment of the invention, the implantable device may comprise a power source, or may be adapted to electrically couple with an external power source for powering the device.

Delivering electrical power to medical implants for powering or communication is a topic which is well-described in literature.

Comprehensive reviews of power aspects for implantable medical devices are given in B. A. Achraf, A. B. Kouki and C. Hung, "Power Approaches for Implantable Medical Devices," sensors, no. 28889-28914; doi:10.3390/s151128889, 2015, J. Lee, J. Jang and Y.-K. Song, "A review on wireless powering schemes for implantable microsystems in neural engineering applications," Biomed Eng Letters, no. DOI 10.1007/s13534-016-0242-2, pp. 6:205-215, 2016, A. Kim, M. Ochoa, R. Rahim and B. Ziaie, "New and Emerging Energy Sources for Implantable Wireless Microdevices," IEEE: SPECIAL SECTION ON NANOBIOSENSORS, no. 10.1109/ACCESS.2015.2406292, 2014, and K. N. Bocan and E. Sejdi'c, "Adaptive Transcutaneous Power Transfer to Implantable Devices: A State of the Art Review," sensors, vol. 16, no. doi:10.3390/s16030393, p. 393, 2016.

Any of these solutions may be used to provide power or a communications channel to the implantable device 12, and some approaches will be discussed below.

A first approach is to provide a wired power source as part of the implantable device. A wired power source may be an ordinary battery (non-rechargeable or rechargeable), directly connected to the implantable device or to its operating electronics. However, since implantable devices usually will be worn over a long period of time, a high capacity and high energy density battery would be of benefit. The power density of (re-chargeable) batteries is expected to grow further making them increasingly suitable for long term monitoring functions.

Instead of conventional batteries, bio-fuel cells or nuclear batteries may be applicable. Another alternative power source, which is very similar to a battery, is a super capacitor, which is a capacitor having an extremely high capacitance and a very low self-discharge characteristic.

Energy harvesters may instead be used to operate any implantable device. Accordingly a power generator could for example be operated by human body energy such as motion of an extremity but also motion of an inner organ or any dynamics resulting from a fluid flow (blood in an artery) or gas (air in a lung). The power generator may be able to store energy in a super capacitor or re-chargeable battery, and/or be able to directly operate an implant.

An energy harvester does not necessarily need to be in close vicinity to the implantable device itself but could also be spatially separated. A wired connection may be used between them. Also in the field of energy harvesters, efforts are being made to make them smaller and more efficient in order to make them more attractive as an internal (and everlasting) energy source for medical devices.

Wireless energy transmission systems may be classified according to the physical coupling mechanism, which can be either capacitive, inductive (magnetic) or electromagnetic. All three mechanisms have their own pros and cons and preferred applications. In general, the performance of each approach depends very much on specific boundary conditions such as e.g. the size of the transmitter- and receiver-element (which can be a plate, an inductor or an antenna) and the distance and medium between both elements, as well as their orientation with respect to each other.

An additional advantageous feature of all wireless power systems is the intrinsic ability of a bidirectional data communication between a transmitter and a receiver.

In applications where low energy levels at short distances need to be transmitted, capacitive coupling may be used. Low to medium power levels at medium to long range may be preferably realized via an electromagnetic coupling. Highest power levels at short distances may be transmitted via an inductive coupling, making use of magnetic fields.

A most basic approach only enables sensor data to be gathered when the external controller is present, in particular if wireless power transfer is used to provide the energy needed for actuation. However, using such a wireless powering technique would not necessarily imply the need to wear such a transmitter continuously to perform the intended use of the implant. For example, an implant may only need to be operated during certain treatments (in e.g. a hospital) or it may only need to be activated at predefined moments in time (e.g. morning, afternoon, evening).

An alternative use case would be to use such a wireless transmitter overnight, to charge an implanted power source, which would be used to operate an implant during the day. This is a hybrid approach where there is a local energy supply so sensor data can be gathered and stored in memory without an external controller in place, but it has a short duration so needs recharging periodically.

The implanted wireless receiver unit and the implanted sensor-actuator may be spatially separated from each other. For example, the receiving element, e.g. a receiver inductance may be located directly underneath the skin, in order to realize a strong coupling between the transmitter and receiver and thus to maximize the energy transmission efficiency and to minimize the charging time of an implanted battery. Of course, this would require a more involved implantation procedure than if the implanted elements are fully integrated into e.g. an artificial valve or stent (or other support structure).

There are also options which do not rely on electrical energy to realize a wireless energy transmission system, in particular making use of optical, ultrasonic or mechanical pressure waves.

As discussed above, the actuator is be implemented using an electroactive polymer (EAP) device. EAPs are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications. An EAP device can be used in any application in which a small amount of movement of a component or feature is desired, based on electric actuation or for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can again be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

For the sensing functionality, the use of capacitance change is one option, in particular in connection with an ionic polymer device. For field driven systems, a capacitance change can also be measured directly or by measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O₂, NO₂), making CNTs usable as gas detectors.

Sensing may also be based on force measurements and strain detection. Dielectric elastomers, for example, can be easily stretched by an external force. By putting a low voltage on the sensor, the strain can be measured as a function of voltage (the voltage is a function of the area).

As discussed above, embodiments make use of a controller for interpreting the sensing signals and driving the actuator. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An implantable device (12) comprising:
a support structure (16);
an actuator (18) comprising an electroactive polymer material, the actuator being mounted to the support structure and wherein the actuator has a direction of actuation;
a sensing means adapted to sense an external force being exerted in a direction opposing said direction of actuation or in said direction of actuation;
a controller (28) for controlling actuation of the actuator and receiving signals from the sensing means, the controller adapted to:
interpret signals from the sensing means to monitor said force over time; and
drive the actuator to actuate at a moment in time when force opposing the direction of actuation is sensed to be at its lowest within a given time window or force in the direction of actuation is sensed to be at its highest within a given time window.

2. An implantable device (12) as claimed in claim 1, wherein the external force is periodic and wherein said time window is a single cycle period of the periodic force.

3. An implantable device (12) as claimed in claim 1 or 2, wherein sensing the force being exerted in a direction toward the actuator comprises sensing a force being applied to at least a region of the actuator.

4. An implantable device (12) as claimed in any preceding claim, wherein the actuator is a sensor-actuator, the sensor-actuator providing the sensing means.

5. An implantable device (12) as claimed in any preceding claim, wherein the sensing means comprises a sensor element, being mounted to the support structure.

6. An implantable device (12) as claimed in any preceding claim, wherein the implantable device is for exerting a force against an internal bodily element.

7. An implantable device (12) as claimed in claim 6, wherein the sensing means is adapted in use to sense an external force being exerted in a direction toward the actuator by said internal body element.

8. An implantable device (12) as claimed in claim 6 or 7, wherein:
the actuator is arranged to adjust a dimension of said internal bodily element; or
the actuator is for positioning within a bodily chamber or conduit, and is arranged in use to permit manipulation of a fluid flow through said chamber or conduit.

9. An implantable device (12) as claimed in any of claims 6-8, wherein at least a part of the actuator is adapted in use to rest against said internal bodily element, and wherein the sensing means is adapted in use to sense a force exerted by the bodily element on the actuator.

10. An implantable device (12) as claimed in any preceding claim, wherein the device comprises an adaptive diameter ring for adjusting an internal dimension of an internal bodily element, the actuator being arranged such that actuation of the actuator changes a diameter of the ring for effecting said adjustment.

11. An implantable device (12) as claimed in claim 10, wherein the adaptive diameter ring comprises an annular arrangement of actuators which at least partially define the ring, the actuators being adapted to deform in a radial direction upon actuation to thereby adjust the diameter of the ring, and optionally wherein said external force is a force exerted toward the actuators in a radial direction.

12. An implantable device (12) as claimed in claim 10 or 11, wherein the adaptive diameter ring is for extending around a blood vessel, for adjusting an internal dimension of the blood vessel, and optionally wherein the sensing means is for sensing a force exerted in a direction radially outward of the vessel, by blood within the vessel or by a wall of the vessel.

13. An implantable device (12) as claimed in claim 10 or 11, wherein the ring is for positioning around a chamber of a heart for adjusting in use an internal dimension of said chamber, and optionally wherein the sensing means is adapted to sense a force exerted in a direction outward of said chamber by a wall of the chamber.

14. An implantable device (12) as claimed in any of claims 10-13, wherein the device comprises a prosthetic valve for a blood vessel or for the heart, the adjustable diameter ring forming at least part of an outer radial wall of said valve.

15. A method of controlling an implantable device (12), the implantable device comprising
a support structure (16),
an actuator (18) comprising an electroactive polymer material, the actuator being mounted to the support structure and wherein the actuator has a direction of actuation;
a sensing means adapted to sense an external force being exerted in a direction opposing said direction of actuation or in said direction of actuation;
a controller (28) for controlling actuation of the actuator and receiving signals from the sensing means, the controller adapted to:
the method comprising:
interpreting signals from the sensing means to monitor said force over time; and
driving the actuator to actuate at a moment in time when force opposing the direction of actuation is sensed to be at its lowest within a given time window or force in the direction of actuation is sensed to be at its highest within a given time window.
